(19) Europäisches Patentamt European Patent Office Office européen des brevets

(11) **EP 3 415 900 B1**

(12) **EUROPEAN PATENT SPECIFICATION**

(45) Date of publication and mention of the grant of the patent:
**20.07.2022 Bulletin 2022/29**

(21) Application number: **16889905.2**

(22) Date of filing: **14.11.2016**

(51) International Patent Classification (IPC):
*G01N 27/04* (2006.01)     *G01N 27/22* (2006.01)
*G01N 33/86* (2006.01)

(52) Cooperative Patent Classification (CPC):
**G01N 33/4905; G01N 33/86**

(86) International application number:
**PCT/JP2016/083660**

(87) International publication number:
**WO 2017/138204 (17.08.2017 Gazette 2017/33)**

(54) **SAMPLE FOR MEASURING ELECTRICAL CHARACTERISTICS, DEVICE FOR MEASURING ELECTRICAL CHARACTERISTICS, AND METHOD FOR MEASURING ELECTRICAL CHARACTERISTICS**

PROBE ZUR MESSUNG ELEKTRISCHER EIGENSCHAFTEN, VORRICHTUNG ZUR MESSUNG ELEKTRISCHER EIGENSCHAFTEN UND VERFAHREN ZUR MESSUNG ELEKTRISCHER EIGENSCHAFTEN

ÉCHANTILLON POUR MESURER DES CARACTÉRISTIQUES ÉLECTRIQUES, DISPOSITIF DE MESURE DE CARACTÉRISTIQUES ÉLECTRIQUES, ET PROCÉDÉ DE MESURE DE CARACTÉRISTIQUES ÉLECTRIQUES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **10.02.2016 JP 2016023555**

(43) Date of publication of application:
**19.12.2018 Bulletin 2018/51**

(73) Proprietor: **Sony Group Corporation Tokyo 108-0075 (JP)**

(72) Inventors:
• **MACHIDA Kenzo**
 **Tokyo 108-0075 (JP)**
• **OMORI Shinji**
 **Tokyo 108-0075 (JP)**
• **HAYASHI Yoshihito**
 **Tokyo 108-0075 (JP)**
• **BRUN Marcaurele**
 **Tokyo 108-0075 (JP)**

(74) Representative: **D Young & Co LLP
120 Holborn
London EC1N 2DY (GB)**

(56) References cited:
WO-A1-2004/109277    WO-A1-2008/010058
WO-A1-2015/098473    JP-A- 2008 509 924
JP-A- 2015 010 939

• SASCHA MEYER DOS SANTOS ET AL: "A novel [mu]-fluidic whole blood coagulation assay based on Rayleigh surface-acoustic waves as a point-of-care method to detect anticoagulants", BIOMICROFLUIDICS, vol. 7, no. 5, 1 September 2013 (2013-09-01), page 056502, XP55514430, DOI: 10.1063/1.4824043

EP 3 415 900 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

[Technical Field]

The present technique relates to a method of verifying a measurement accuracy of an electric characteristic measuring apparatus as defined in the appended claims.

[Background Art]

Against a risk of thrombosis, an anti-platelet aggregation drag or an anticoagulant is medicined in a preventive manner. In this preventive administration, there is a side effect that in the case where a dosage is excessive, a bleeding risk increases. For the purpose of obtaining a sufficient preventive effect while this side effect is prevented, it is carried out to evaluate a blood coagulation ability of an administered person in a timely fashion.

In recent years, the development of the technique for conveniently and accurately evaluating the degree of the coagulation of the blood has been progressed. For example, PTL 1 discloses the technique for acquiring information associated with the blood coagulation from a dielectric constant of the blood. Thus, PTL 1 describes "a blood coagulation system analyzing apparatus having a pair of electrodes, application means for applying an alternating-current (AC) voltage across the pair of electrodes at a given time interval, measurement means for measuring a dielectric constant of blood arranged between the paired electrodes, and analysis means for analyzing the degree of an action of a blood coagulation system by using the dielectric constant of the blood which is measured at the time interval after an anticoagulant effect acting on the blood is solved."

[Citation List]

[Patent Literature]

[PTL 1]

[PTL 2]
WO 2008/010058 A1 (UNIVERSAL BIOSENSORS PTY LTD [AU]; CHATELIER RONALD C [AU]; NEWMAN PET) 24 January 2008 discloses a method for electrochemically monitoring the mobility of particles in a fluid in response to an external fluid.

[PTL 3]
SASCHA MEYER DOS SANTOS ET AL: "A novel [mu]-fluidic whole blood coagulation assay based on Rayleigh surface acoustic waves as a point-of-care method to detect anticoagulants", BIOMICROFLUIDICS, val. 7, no. 5, 1 September 2013, page 056502, XP55514430, DOI: 10.1063/1.4824043 discloses a micro-fluidic universal coagulation test employing surface acoustic waves.

[PTL 4]
WO 2004/109277 A1 (HALL EFFECT TECHNOLOGIES LTD [GB]; UNIPATH L TO [GB]; FULLER JONATHAN A) 16 December 2004 discloses a method and apparatus for determining the coagulation states of a liquid.

[Summary]

[Technical Problem]

Here, such an apparatus for measuring the electric characteristics of a blood sample as disclosed in PTL 1 involves the actual situation that before a blood sample from a subject being tested is measured, an attempt to confirm the measurement accuracy is not carried out. If there is some abnormality in the apparatus for measuring the electric characteristics of the blood sample or the measurement procedure thereof, and thus there is encountered the situation in which the accurate measurement could not be carried out, then, not only the measurement itself becomes useless, but also the blood sample collected from the subject being tested becomes useless.

In the light of the foregoing, it is a principal object of the present technique to provide a technique which, in measurement of electric characteristics of a blood sample, is useful in verification of a measurement accuracy before the measurement thereof.

[Solution to Problem]

The invention is set out in the appended claims.

[Advantageous Effect of Invention]

According to the present technique, in the measurement of the electric characteristics of a blood sample, the measurement accuracy can be verified before the measurement.

It should be noted that the effect described here is not necessarily limited and any of effects described in the present technique may also be offered.

[Brief Description of Drawings]

[FIG. 1]

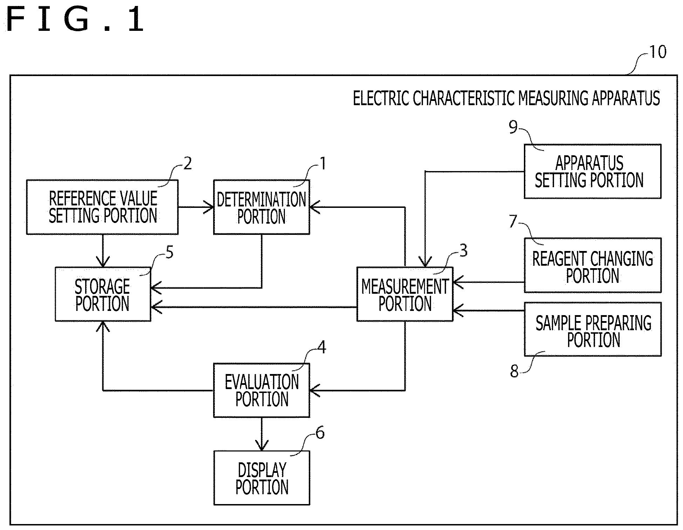

FIG. 1 is a block diagram depicting a schematic configuration of an example of an embodiment of an electric characteristic measuring apparatus 10.
[FIG. 2]

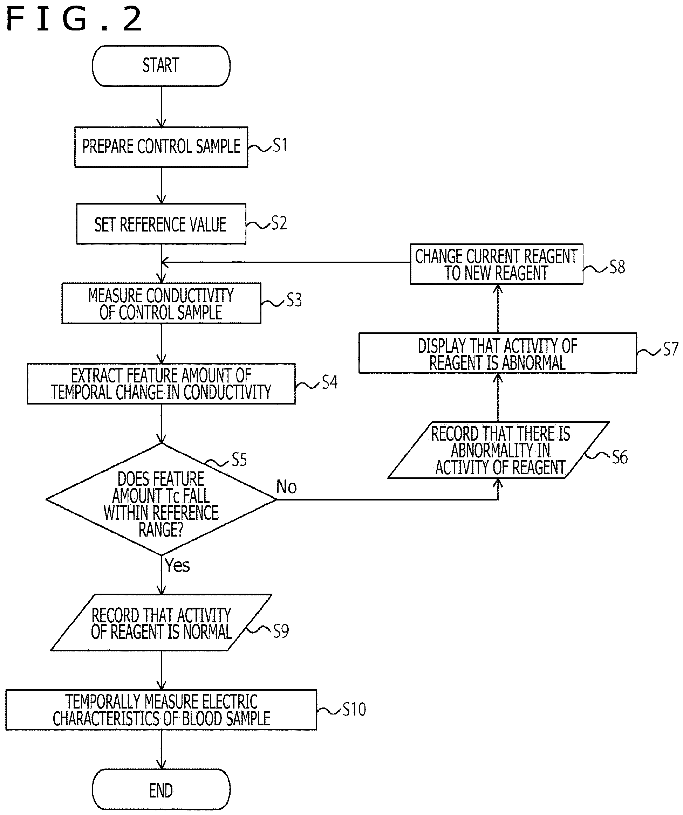

FIG. 2 is a flow chart depicting Processing Example 1 carried out in the apparatus 10.
[FIG. 3]

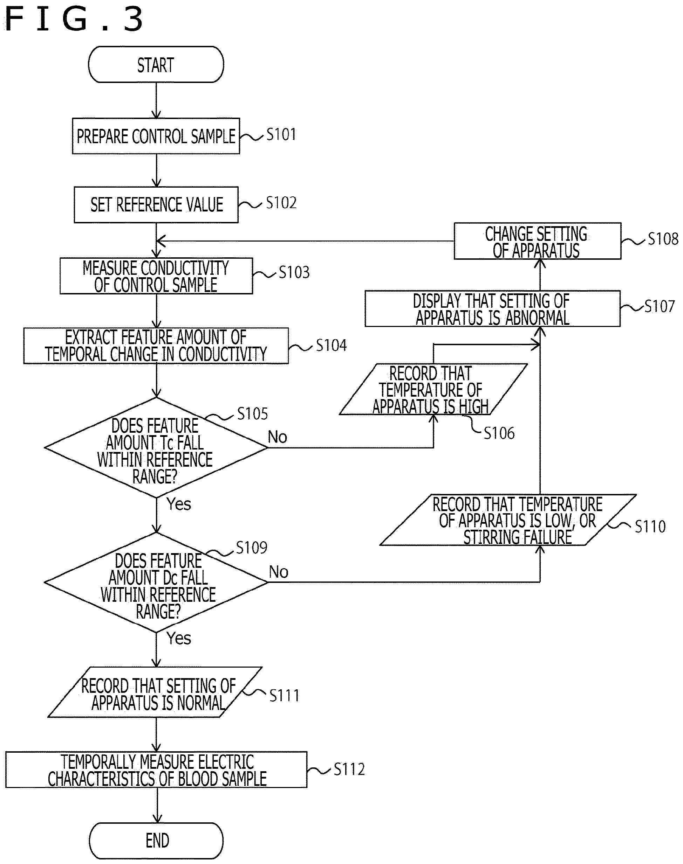

FIG. 3 is a flow chart depicting Processing Example 2 carried out in the apparatus 10.
[FIG. 4]

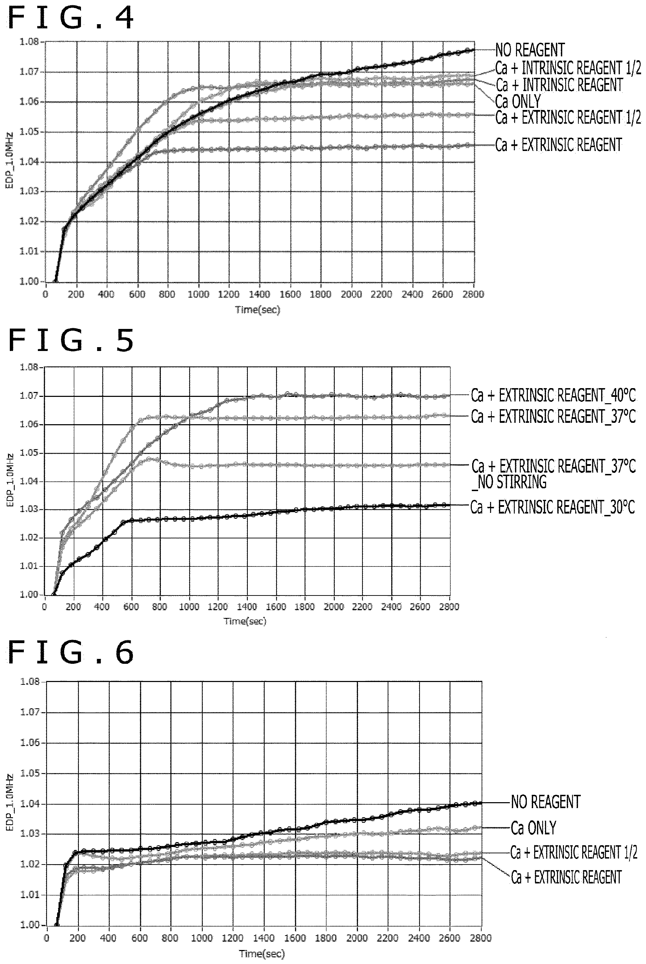

FIG. 4 is a graphical representation substituted for a drawing depicting measurement results of a temporal change in conductivity of Example 1.
[FIG. 5]
FIG. 5 is a graphical representation substituted for a drawing depicting measurement results of a temporal change in conductivity of Example 2.
[FIG. 6]
FIG. 6 is a graphical representation substituted for a drawing depicting measurement results of a temporal change in conductivity of Comparative Example 1.

[Description of Embodiment]

Hereinafter, a preferred embodiment of the present technique will be described with reference to the drawings. It should be noted that an embodiment which will be described below represents an example of a representative embodiment of the present technique, and the scope of the present technique is not narrowly interpreted by the representative embodiment. The description will be given in accordance with the following order.

1. Sample for Measurement of Electric Characteristics

　　(1) Liquid containing blood coagulation factor
　　(2) Micro particles
　　(3) Arbitrary component

2. Electric Characteristic Measuring Apparatus 10

　　(1) Determination portion 1
　　(2) Reference value setting portion 2
　　(3) Measurement portion 3
　　(4) Evaluation portion 4
　　(5) Storage portion 5
　　(6) Display portion 6
　　(7) Reagent changing portion 7
　　(8) Sample preparing portion 8
　　(9) Apparatus setting portion 9

　　　[Processing Example 1 carried out in apparatus 10]

[Processing Example 2 carried out in apparatus 10]

3. Electric Characteristic Measuring Method

        (1) Determination process
        (2) Reference value setting process
        (3) Preparation process
        (4) Measurement process

1. Sample for Measurement of Electric Characteristics

A sample for measurement of electric characteristics not falling within the scope of the claims is used in measurement of electric characteristics, and contains at least a liquid containing a blood coagulation factor, and micro particles. The sample for measurement of electric characteristics is suitably used in the measurement of the electric characteristics of a blood sample as a measurement object and is useful in verification of the measurement accuracy. From this fact, hereinafter, the sample for measurement of electric characteristics will be referred to as "a control sample" in some cases.

Using the control sample results in that a state (for example, activity or the like) of a reagent used at the time of measurement of the electric characteristics can be verified. For this reason, the control sample is used as a so-called sample for Quality Control (hereinafter, referred to as "QC" as well), and thus can be used for management of experimental accuracy.

The reagent is not especially limited and, for example, an intrinsic stimulation reagent, an extrinsic stimulation reagent or the like in the case where a blood sample is used as a measurement object. In addition, in the present technique, these reagents are added to the control sample, and both of them are mixed with each other within a cartridge type measuring container, and the electric characteristics of the resulting reagent are measured, thereby enabling the QC of the reagent concerned to be carried out. It should be noted that, in the present technique, the aspect in which these reagents are added to the object sample is not especially limited, and thus an aspect may also be available in which these reagents are previously contained in the cartridge type measuring container.

In addition, the control sample is more suitably used in an apparatus (electric characteristic measuring apparatus not falling within the scope of the claims) for measuring the electric characteristics of the blood sample as the measurement object. Using the control sample in the electric characteristic measuring apparatus for the blood sample results in that the state of that apparatus can be verified. As a result, the control sample can also be used as the sample for QC for management of the quality of the apparatus. The state of the apparatus concerned, for example, contains the temperature setting, the stirring function, the electric contact, an electric measurement circuit, and the like.

The electric characteristic measuring apparatus is preferably an electric characteristic measuring apparatus for temporally measuring the electric characteristics at an arbitrary frequency with respect to a blood sample as a measurement object. The electric characteristic measuring apparatus may be provided with a function of evaluating a state of the blood sample as the measurement object, or may be provided with a function of analyzing the state of the blood sample on the basis of the measured electric characteristics.

In addition, the control sample has an advantage that it can be conveniently and inexpensively produced because it does not take time and effort in the production as compared with past samples for the QC.

The electric characteristic measuring apparatus which can suitably use the control sample, a concrete technique for carrying out the QC by using the control sample, and the like will be described later. Firstly, a description will be given with respect to a constitution of the control sample.

(1) Liquid containing blood coagulation factor

The sample for measurement of the electric characteristics contains the blood coagulation factor, and when being mixed with a liquid which will be described later, the electric characteristics of the liquid described above containing the blood coagulation factor can be temporally changed. The blood coagulation factor is not especially limited and, for example, includes calcium ($Ca^{2+}$), fibrinogen, fibrin, prothrombin, thrombin, thromboplastin or the like.

Although the origin of the blood coagulation factor is not especially limited, in the present technique, the blood coagulation factor derived from the freeze-dried plasma is preferably used. Use of the blood coagulation factor derived from the freeze-dried plasma results in that the preservation stability of the control sample becomes satisfactory.

Although the liquid described above is not especially limited, a dispersing liquid for dispersing the blood coagulation factor and/or micro particles which will be described later is used as the liquid described above. The dispersing liquid is used as the liquid, which results in that, for example, the liquid in which the blood coagulation factor is dispersed, or the liquid in which the micro particles are dispersed can be prepared prior to the use of the control sample, and thus the convenience of a user is enhanced. In addition, in this case, the control sample can be made kit type one which will be described later.

The dispersing liquid described above is not especially limited as long as it can disperse the blood coagulation factor and/or the micro particles which will be described later. However, the solution which can make the salt concentration and pH close to the plasma and does not have the reactivity with the blood coagulation factor or the micro particles is preferable as the dispersing liquid. Specifically, the dispersing liquid includes a buffer solution such as phosphate buffered saline (hereinafter, referred to as "PBS" as well), a diluted solution such as a saline solution, or the like.

In addition, in the present technique, a surfactant or the like may also be added to the liquid in the range in which there is no influence exerted on the blood coagulation reaction, thereby enhancing the dispersiveness of the micro particles which will be described later to the liquid described above.

In addition, in the present technique, the blood coagulation factor and/or the micro particles which will be described later may be previously dispersed in the control sample by the dispersing liquid described above. As a result, the time and effort of the user in the sample preparation stage are saved, thereby enhancing the convenience of the user. In addition, in this case as well, the control sample can be made kit type one which will be described later.

It should be noted that in the present technique, the liquid containing therein the blood coagulation factor, for example, is the plasma and the artificial plasma can also be used as the plasma.

(2) Micro particles

The sample for measurement of electric characteristics contains the micro particles (referred to as "microbeads" as well). The micro particles described above are not especially limited and, for example, includes micro particles or the like including a material such as synthetic polymer such as vinyl polymer, thermosetting polymer, polycondensation polymer, polyaddition polymer or olefin-based polymer, or natural-based polymer. In the present technique, among them, especially, the micro particles which have the dispersiveness to the water, and in which an influence on the coagulation of the plasma is small are preferable.

In addition, a moving velocity (v) of the micro particles described above is expressed by following Numerical Expression (1) based on the Stokes' law.

[Math. 1]

$$v = (\rho_B - \rho_L)gd^2/18\eta \qquad \cdots (1)$$

$\rho_B$: density of micro particle
$\rho_L$: density of plasma
d: diameter of micro particle
$\eta$: viscosity of plasma
g: acceleration of gravity

However, in derivation of Numerical Expression (1) described above, a resistance coefficient of the micro particles is approximated as $C_d = 24/Re^*$ ($Re^*$: Reynolds number of the micro particles).

According to the law described above, for example, since the density of the plasma is approximately 1.03 kg/m$^3$, the micro particles each having the density smaller than that density rise, while the micro particles each having the density larger than that density sink. The inventors of the present application have found out that the state of the reagent, the state of the electric characteristic measuring apparatus, or the like can be checked by observing the difference in density to be made useful in the QC. In other words, the present technique is characterized in that the density of each of the micro particles is made different from the density of the liquid containing therein the blood coagulation factor. It should be noted that the "density" in the present technique means a "mass density."

The difference in density described above, for example, can be confirmed by observing the temporal change in conductivity or dielectric constant between the liquid containing therein the blood coagulation factor, and the micro particles. In particular, an increase in conductivity or dielectric constant is easy to confirm, which is suitable.

In addition, the moving velocity (v) of the micro particles depends on the diameter of each of the micro particles as well as the difference in density. Then, for making the measurement falls within a suitable measurement time, it is only necessary to adjust the density and size of each of the micro particles.

The density of each of the micro particles is not especially limited. For example, when the consideration is made with the density of plasma (approximately 1.03 kg/m$^3$) as a reference, the micro particles each having the density smaller than that density, or the micro particles each having the density larger than that density can be suitably selected.

The micro particles each having the density smaller than the density of the plasma (that is, the particles of a type in which they rise with respect to the plasma), for example, include thermal expansion micro sphere: Matsumoto Microsphere series (manufactured by Matsumoto Yushi-Seiyaku Co., Ltd.), ADVANCELL EM (manufactured by SEKISUI CHEMICAL CO., LTD.), KUREHA Microsphere (manufactured by KUREHA CORPORATION), and the like as the articles on the market. In addition thereto, the micro particles each having the density smaller than the density of the plasma include polymer-based micro particles including polyethylene, polypropylene and the like.

The micro particles each having the density larger than the density of the plasma (that is, the particles of a type in which they sink with respect to the plasma), for example, include polymer-based micro particles including polystyrene, PMMA (Poly Methyl Methacrylate), polyacryl, polyacrylamide, nylon, polyurethane, polyurea, epoxy, polyether sulfone, poly phenylene sulfide, polyamide, polyamide imide, cellulose, sephadex, cross-linked agarose, and the like.

Although the size of each of the micro particles is not especially limited, for example, the micro particles having an average particle diameter of 1 to 100 $\mu$m, preferably an average particle diameter of 5 to 50 $\mu$m, can be used. In addition, in the present technique, microcapsule type micro particles enclosing hydrocarbon as a thermal expansion agent or the like can be used.

Although the density of each of the micro particles is not especially limited, in the case where the liquid containing the blood coagulation factor described above is used as the plasma, the density of the plasma is approximately 1.03 kg/m$^3$. Therefore, in the present technique, the micro particles each having the density smaller than that density (for example, 1.02 kg/m$^3$ or less, preferably, 1.01 kg/m$^3$ or less) or the micro particles each having the density larger than that density (for example, 1.04 kg/m$^3$ or more, preferably, 1.05 kg/m$^3$ or more) can be suitably, selectively used.

(3) Arbitrary component

The control sample may contain therein a preservative solution or the like which can be suitably used for the preservation of the blood coagulation factor in addition to the components described above. One kind or two or more kinds of known preservative solution can be selectively used as the preservative solution unless they injure the effects of the present technique. The preservative solution, for example, includes an ACD solution containing a given amount of sodium citrate hydrate, a given amount of citric acid hydrate, and a given amount of glucose, a CPD solution containing a given amount of sodium citrate hydrate, a given amount of citric acid hydrate, a given amount of glucose, and a given amount of sodium dihydrogen phosphate, a MAP solution, an AIS solution containing adenine, inosine, and sucrose, and the like. The preservative solutions on the market can be used as these preservative solutions. It should be noted that the MAP solution is a preservative solution containing therein a given amount of D-mannitol, a given amount of adenine, a given amount of sodium dihydrogen phosphate, a given amount of sodium citrate hydrate, a given amount of citric acid hydrate, a given amount of glucose, and a given amount of sodium chloride.

It should be noted that the sample for measurement of electric characteristics can also be made kit type one containing the blood coagulation factor which is previously dispersed by the dispersing liquid and in this state is subjected to the freeze-drying to be preserved, and the micro particles, kit type one containing the micro particles which are previously dispersed to the dispersing liquid and in this state is preserved, and the liquid containing the blood coagulation factor, or kit type one containing the liquid containing the blood coagulation factor and the micro particles which are mixed with each other, and the dispersing liquid. In addition, in the kit concerned, the liquid itself containing therein the blood coagulation factor may be subjected to the freeze-drying or the like to be preserved in this state, the liquid containing the blood coagulation factor and the micro particles may be mixed with each other to be temporarily subjected to the freeze-drying or the like to be preserved in this state.

Moreover, the arbitrary components or the like described above may also be contained in the kit described above. The control sample is made the kit type one, resulting in that when the control sample is used in measurement of the electric characteristics, for example, the control sample become easy to adjust so as to obtain the temporal change of the electric characteristics in response to the apparatus used in that measurement. In addition, the convenience for the user is enhanced.

2. Electric Characteristic Measuring Apparatus 10

The electric characteristic measuring apparatus 10 is provided with at least a determination portion 1. FIG. 1 is a block diagram depicting a schematic configuration of an example of the electric characteristic measuring apparatus 10. The electric characteristic measuring apparatus 10 may be provided with a reference value setting portion 2, a measurement portion 3, an evaluation portion 4, a storage portion 5, a display portion 6, a reagent changing portion 7, a sample preparing portion 8, an apparatus setting portion 9, and the like as may be necessary in addition to the determination portion 1. It should be noted that since the sample for measurement of electric characteristics is similar to that described above, a description thereof is omitted here.

(1) Determination portion 1

The determination portion 1 determines the measurement accuracy on the basis of a measured value based on the electric characteristics temporally measured by using the sample for measurement of electric characteristics, and a reference value previously given.

In the case where the determination result of the measurement accuracy by the determination portion 1 represents that the measurement accuracy is low, for example, the measured value is abnormal, the user of the apparatus 10 can confirm whether or not there is the abnormality in the apparatus, there is the abnormality in the sample for measurement of electric characteristics, and so forth.

In addition, in the present technique, in the case where the determination result represents that the measurement accuracy is low, the determination portion 1 may execute processing or the like in which the determination result is left in a log file or the like.

Before the measurement is carried out with respect to the blood sample by using the electric characteristic measuring apparatus 10 provided with the determination portion 1, the electric characteristics are temporally measured with respect to the control sample, resulting in that the measurement accuracy of the apparatus 10 concerned can be confirmed prior to the measurement about the blood sample. Therefore, the highly reliable measurement data can be acquired with respect to the blood sample. It should be noted that the reference value may have a numerical value range having a given width. In addition, the reference value can be stored in the storage portion 5 which will be described later, an external memory or the like.

The electric characteristics used in the determination portion 1 is data which is measured by using the measurement portion 3 provided inside the electric characteristic measuring apparatus 10 or the external measurement apparatus. The electric characteristics, for example, include impedance, conductance, admittance, capacitance, a dielectric constant, conductivity, a phase angle, quantities obtained by subjecting these characteristics to the electric quantity conversion, and the like. The electric characteristics used in the determination portion 1 can be used in combination of one kind or two or more kinds of these characteristics.

A determination method, more specifically, includes a method in which, for example, data exhibiting a change in measured electric characteristics of the control sample, and a reference value previously given are compared with each other, and presence or absence of a problem in the measurement accuracy is determined on the basis of a difference between them, and the like.

With regard to this method, a given feature amount is extracted from the change in conductivity or dielectric constant with the conductivity or dielectric constant as an index, and whether or not a value in the feature amount falls within a given range is determined.

Although the given feature amount which is to be extracted from the conductivity or the dielectric constant is not especially specified, the given feature amount is set at a time until the change in the conductivity or the dielectric constant is suppressed, or an amount of change in conductivity or dielectric constant until this time. A method of calculating these feature amounts, for example, includes a method in which in a graph expressing a temporal change in conductivity or dielectric constant, (1) at a certain frequency, a difference between the adjacent measured points is obtained, and a point is obtained at which the difference becomes a given threshold value or less, (2) at a certain frequency, first derivation is calculated, and a point is obtained at which the first derivation becomes a previously set threshold value or less, or (3) at a certain frequency, an intersection point or the like between the approximation straight line for a measurement point of a portion in which a change in conductivity or dielectric constant is large, and the approximation straight line for a measurement point after a change in the conductivity or dielectric constant cannot be observed is obtained and calculated, and the like.

In the present technique, the "feature amount," for example, means a given value such as a maximum value or a minimum value of the conductivity or the dielectric constant, or a given change rate of a difference between the maximum value and the minimum value of the conductivity or the dielectric constant.

In other words, in the present technique, for example, in the case where the maximum value of the conductivity or the dielectric constant is A, and the minimum value of the conductivity or the dielectric constant is B, it is possible to determine whether or not a difference between A and B falls within ±10%. In addition, it is possible to determine whether or not a time Tc until the change (for example, rising, sinking or the like) in conductivity or dielectric constant is suppressed is within 2000 seconds, preferably within 1600 seconds. Moreover, in the case where the maximum value of the conductivity or the dielectric constant is A, and the minimum value of the conductivity or the dielectric constant is B, it is possible to determine whether or not a change rate between A and B is +5% or more, preferably +6% or more. The setting of such a reference results in that the change in electric characteristics becomes easy to confirm. Therefore, the control sample can be more suitably used as the QC sample.

In the case where the determination result of the measurement accuracy by the determination portion 1 represents that the measurement accuracy is low, for example, the measured value is abnormal, the user of the apparatus 10 can confirm whether or not there is the abnormality in the apparatus, there is the abnormality in the state (for example, the activity or the like) of the sample for measurement of electric characteristics or the reagent used at the time of the measurement of the electric characteristics, and so forth.

The electric characteristics are temporally measured with respect to the control sample before the measurement is carried out with respect to the blood sample by using the electric characteristic measuring apparatus 10 provided with the determination portion 1, resulting in that the measurement accuracy of the apparatus 10 can be confirmed prior to the measurement about the blood sample. Therefore, the highly reliable measurement data can be acquired with respect to the blood sample. It should be noted that the reference value may have a numerical value range having a given width. In addition, the reference value can be stored in the storage portion 5 which will be described later, the external memory or the like.

The electric characteristics used in the determination portion 1 is data which is measured by using the measurement portion 3 provided inside the electric characteristic measuring apparatus 10 or the external measurement apparatus. The electric characteristics, for example, include impedance, conductance, admittance, capacitance, a dielectric constant, conductivity, a phase angle, quantities obtained by subjecting these characteristics to the electric quantity conversion, and the like. The electric characteristics used in the determination portion 1 can be used in combination of one kind or two or more kinds of these characteristics.

(2) Reference value setting portion 2

The electric characteristic measuring apparatus 10 can be further provided with the reference value setting portion 2. The reference value setting portion 2 sets the reference value on the basis of the information storage medium provided for every sample for measurement of electric characteristics. The information storage medium stores the reference value described above. With regard to a more concrete aspect of the reference value setting portion 2, for example, the reference value described above is read out or detected by a reader or the like dedicated to the information storage medium from the medium concerned, thereby setting the reference value.

The electric characteristic measuring apparatus 10 is further provided with the reference value setting portion 2, whereby at the time of the measurement, the data associated with the reference value can be inputted to set the range or the like of a normal value, and the time and effort for inputting the data associated with the reference value existing for every sample for measurement of electric characteristics can be saved. Therefore, the convenience for the user is enhanced. In addition, the occurrence of a human error resulting from the manual input of the reference value can be prevented.

The information storage medium described above, specifically, for example, includes an IC card, an IC tag, a card provided with a barcode or a matrix type two-dimensional code, a paper or a seal on which a barcode or a matrix type two-dimensional code is printed, and the like.

(3) Measurement portion 3

The measurement portion 3 temporally measures the electric characteristics of the control sample and the blood sample at a specific frequency or a specific frequency band. The electric characteristic measuring apparatus 10 may not be provided with the measurement portion 3. In this case, the data obtained through the measurement by the external electric characteristic measuring apparatus can be used. Since concrete examples of the electric characteristics measured by the measurement portion 3 are similar to those described in the determination portion 2 described above, a description thereof is omitted here.

It should be noted that such a configuration may also be adopted that the noise is removed away from the data associated with the temporal change in electric characteristics measured by the measurement portion 3 or the like by a data processing portion provided inside the electric characteristic measuring apparatus 10 or an external data processing apparatus.

In carrying out the measurement, the measurement portion 3 can be provided with one or a plurality of sample holding portions into each of which the control sample or the blood sample is to be injected. The electric characteristic measuring apparatus 10 may not be provided with the sample holding portion. In this case, for example, the measurement portion 3 can also be designed so as to have a form in which a cartridge type measuring container or the like can be installed. In the present technique, as the cartridge type measuring container described above, a container in which electrodes are arranged in a part of the container is preferably used.

In the case where the measurement portion 3 applies an AC voltage across the paired electrodes provided in the sample holding portion to measure the impedance or dielectric constant of the control sample or the blood sample, an impedance analyzer, a network analyzer or the like can also be used as the measurement portion 3.

(4) Evaluation portion 4

The electric characteristic measuring apparatus 10 may be further provided with the evaluation portion 4. The evaluation portion 4 evaluates a state of the blood sample on the basis of the data associated with the temporal change in electric characteristics of the blood sample. The state of the blood sample as the evaluation object, for example, includes the coagulation state of the blood sample, the cohesion state of the components in the blood sample, the state of the sinking or rouleau of the red blood cells, the blood clot degeneration state, and the like. The electric characteristics described above in an arbitrary frequency (for example, a frequency of 1 kHz to 50 MHz) responding to the state of the blood sample which is desired to be predicted or detected can be used as the electric characteristics to be used in the evaluation in the evaluation portion 4.

(5) Storage portion 5

The electric characteristic measuring apparatus 10 may be further provided with the storage portion 5. The storage portion 5 stores therein the determination result in the determination portion 1, the value of the reference value set by the reference value setting portion 2, the measurement result of the control sample or the blood sample obtained through the measurement in the measurement portion 3 or the like, the evaluation results in the evaluation portion 4 described above, and the like. The storage portion 5 is not essential to the electric characteristic measuring apparatus 10. Thus, an external memory may be connected to the electric characteristic measuring apparatus 10. A hard disc or the like, for example, can be used as the storage portion 5.

(6) Display portion 6

The electric characteristic measuring apparatus 10 may be further provided with the display portion 6. The display portion 6 displays thereon the determination result in the determination portion 1, the value of the reference value set by the reference value setting portion 2, the data associated with the temporal change in electric characteristics of the control sample or the blood sample measured by the measurement portion 3 or the like, the evaluation result in the evaluation portion 4 described above, and the like. The display portion 6 is not essential to the electric characteristic measuring apparatus 10. Thus, an external display device may be connected to the electric characteristic measuring apparatus 10.
It should be noted that the electric characteristic measuring apparatus 10 may be utilized as a part of a system connected to a server, a user interface or the like through a network.

(7) Reagent changing portion 7

The electric characteristic measuring apparatus 10 may be further provided with the reagent changing portion 7. In the case where a state of the reagent to be used at the time of the measurement of the electric characteristics is verified by using the control sample, or the like, the reagent changing portion 7, for example, when the state of the reagent is abnormal, discards the abnormal reagent to change the abnormal reagent over to a new reagent. The reagent changing portion 7 is not essential to the electric characteristic measuring apparatus 10, and thus the user himself/herself may execute the processing which should be executed in the reagent changing portion 7. In addition, in the present technique, the determination portion 1, the measurement portion 3 or the like described above may have the function of this reagent changing portion 7.

(8) Sample preparing portion 8

The electric characteristic measuring apparatus 10 may be further provided with the sample preparing portion 8. The sample preparing portion 8 prepares the control sample. The sample preparing portion 8 is not essential to the electric characteristic measuring apparatus 10. Thus, the user himself/herself of the apparatus 10 may execute the processing which should be executed in the sample preparing portion 8. In addition, in the present technique, the determination portion 1, the measurement portion 3 or the like described above may have the function of the sample preparing portion 8.

(9) Apparatus setting portion 9

The electric characteristic measuring apparatus 10 may be further provided with the apparatus setting portion 9. For example, in the case where the state of the apparatus 10 is verified by using the control sample, or the like, when the state of the apparatus 10 is abnormal, the apparatus setting portion 9 changes the abnormal apparatus setting over to new apparatus setting. More specifically, the apparatus setting, for example, includes: in the case where a temperature of the apparatus 10 is a high temperature, the setting temperature is reduced; in the case where a temperature of the apparatus 10 is a low temperature, the setting temperature is raised; in case of a stirring failure, the setting (for example, the number of rotations, the rotation direction, or the like) about the stirring of the apparatus 10 is changed; and the like. The apparatus setting portion 9 is not essential to the electric characteristic measuring apparatus 10. Thus, the user himself/herself of the apparatus 10 may execute the processing which should be executed in the apparatus setting portion 9. In addition, in the present technique, the determination portion 1, the measurement portion 3 or the like described above may have the function of the apparatus setting portion 9.

Hereinafter, a description will be given with respect to concrete examples of the processing executed in the apparatus 10 by exemplifying the case where the conductivity is temporally measured as the electric characteristics at an arbitrary frequency with respect to the control sample.

[Processing Example 1 carried out in apparatus 10]

FIG. 2 is a flow chart depicting Processing Example 1 carried out in the apparatus 10. Firstly, the sample preparing portion 8 prepares the control sample by manipulation of the user (S1). Next, the reference value setting portion 2 sets the reference value described above (S2). Thereafter, the measurement portion 3 measures the temporal change of the conductivity of the control sample (to which the reagent becoming the QC object is added) (S3). Next, the determination portion 1 extracts the feature amount of the temporal change of the conductivity (S4). The feature amount concerned, for example, can include the time Tc until the charge in conductivity is suppressed, and the like. In the case where the feature amount Tc falls within the reference range (for example, within 2000 seconds, preferably within 1600 seconds) (S5), the determination portion 1 records that the activity of the reagent is normal in the log file or the like (S9). After that, the measurement portion 3 temporally measures the electric characteristics of the blood sample (S10), and the processing is ended. On the other hand, in the case where the feature amount Tc does not fall within the reference range (including the case where the feature amount Tc is not detected within the measurement time) (S5), the determination portion 1 records that there is the abnormality in the activity of the reagent in the log file or the like (S6), and the display portion 6 displays therein that the activity of the reagent is abnormal (S7). Thereafter, the reagent changing portion 7 changes the current reagent over to the new reagent by the manipulation of the user (S8).

It should be noted that, in the case where the determination portion 1 makes a determination about the activity of the reagent which is previously contained in the cartridge type measuring container, the reagent changing portion 7 changes the current measuring container over to the new measuring container.

[Processing Example 2 carried out in apparatus 10]

FIG. 3 is a flow chart depicting Processing Example 2 carried out in the apparatus 10. Firstly, the sample preparing portion 8 prepares the control sample by manipulation of the user (S101). Next, the reference value setting portion 2 sets the reference value described above (S102). Thereafter, the measurement portion 3 measures the temporal change of the conductivity of the control sample (S103). After that, the determination portion 1 extracts the feature amount of the temporal change of the conductivity (S104). The feature amount concerned, for example, can include the time Tc until the change in conductivity is suppressed, an amount Dc of change in conductivity until the time Tc concerned, and the like. In the case where the feature amount Tc falls within the reference range (for example, within 1000 seconds) (S105), and the feature amount Dc falls within the reference range (for example, in the case where the maximum value of the conductivity is A and the minimum value of the conductivity is B, the change rate between A and B is +5% or more, preferably +6% or more) (S109), the determination portion 1 records that the setting of the apparatus is normal in the log file or the like (S111). Thereafter, the measurement portion 3 temporally measures the electric characteristics of the blood sample (S112), and the processing is ended. On the other hand, in the case where the feature amount Tc does not fall within the reference range (S105), the determination portion 1 records that the temperature of the apparatus is high in the log file or the like (S106), and the display portion 6 displays thereon that the setting of the apparatus is abnormal (S107). Thereafter, the apparatus setting portion 9 changes the setting of the apparatus by the manipulation of the user (S108). In addition, even in the case where the feature amount Tc falls within the reference range (S105), when the feature amount Dc does not fall within the reference range (S109), the determination portion 1 records that the temperature of the apparatus is low or there is the stirring failure (S110), and the display portion 6 displays thereon that the setting of the apparatus is abnormal (S107). After that, the apparatus setting portion 9 changes the setting of the apparatus by the manipulation of the user (S108).

It should be noted that, although in Processing Example 2 depicted in FIG. 3, it is set as the reference that the feature amount Tc falls within the given value, the present technique is by no means limited thereto, and thus it may be set as the reference that the feature amount Tc is the given value or more. In addition, although it is also set as the reference that the feature amount Dc is the given value or more, the present technique is by no means limited thereto, and thus it may be set as the reference that the feature amount Dc falls within the given value.

The control sample is used in such a manner, resulting in that the state of the reagent or the state of the apparatus can be confirmed, and the various kinds of QCs can be efficiently carried out. It should be noted that although in two Processing Examples described above, in the electric characteristic measuring method according to the present technique, the conductivity is used as the index, other indices such as the dielectric constant can also be used.

It should be noted that the functions carried out in the respective portions of the electric characteristic measuring apparatus 10 can be stored as a program in a personal computer or a hardware resource including a control portion including a CPU or the like, a recording medium (such as a non-volatile memory (such as a USB memory), an HDD, or a CD), and the like, thereby being realized by the personal computer or the control portion.

3. Electric Characteristic Measuring Method

The electric characteristic measuring method according to the present technique is a method in which before the electric characteristics of the blood sample are temporally measured, the electric characteristics of the sample for measurement of electric characteristics are temporally measured. With this method, the measurement accuracy of the electric characteristics can be verified by using the control sample prior to the measurement about the blood sample. Therefore, with regard to the blood sample which is to be measured in electric characteristics after the verification, the measurement data having the high reliability can be acquired. The electric characteristic measuring method according to the present technique, for example, is embodied by the processing as depicted in Processing Examples 1 and 2 (FIG. 2 and FIG. 3) described above. It should be noted that since the sample for measurement of electric characteristics is similar to that described above, a description thereof is omitted here.

An electric characteristic measuring apparatus such as a dielectric coagulometer is preferably used in the measurement about the control sample and the blood sample.

(1) Determination process

With the electric characteristic measuring method according to the present technique, there is carried out a determination process for determining the measurement accuracy on the basis of the measured value based on the electric characteristics which are temporally measured by using the sample for measurement of electric characteristics, and the reference value previously given. As a more concrete aspect of the determination process, for example, the activity of the reagent used at the time of the measurement, or the abnormality of the apparatus is detected by the determination portion 1 described above. More specifically, for example, the pieces of processing depicted in S4 to S6 and S10 of FIG. 2, and S104 to S106 and S109 to S111 of FIG. 3 are executed.

(2) Reference value setting process

With the electric characteristic measuring method according to the present technique, the reference value described above is previously given for every sample for measurement of electric characteristics described above, and thus the measurement method concerned further includes a reference value setting process for setting the reference value before the determination process. The reference value setting process is further included, resulting in that at the time of the measurement, the data associated with the reference value can be inputted, thereby setting a range for a normal value. As a more concrete aspect of the reference value setting process, for example, the reference value is set by the reference value setting portion 2 described above. More specifically, for example, the pieces of processing depicted in S2 of FIG. 2, and S102 of FIG. 3 are executed.

(3) Preparation process

The electric characteristic measuring method according to the present technique may further include a preparation process for preparing the control sample. The preparation process is further included, resulting in that the degree of the temporal change of the electric characteristics about the control sample can be controlled, and thus the data associated with the temporal change of the electric characteristics closer to that of the blood sample can be obtained. More specifically, for example, the pieces of processing depicted in S1 of FIG. 2, and S101 of FIG. 3 are executed.

In the present technique, the preparation process may be carried out by the user himself/herself, or may be carried out by the sample preparing portion 8 of the apparatus 10 described above.

The preparation of the control sample, for example, includes: dispersing the blood coagulation factor and/or the micro particles by the liquid; adding the reagent described above to the sample for measurement of electric characteristics; and so forth.

(4) Measurement process

The electric characteristic measuring method according to the present technique further includes a measurement process for temporally measuring the electric characteristics of the blood sample. More specifically, for example, the pieces of processing depicted in S3 and S10 of FIG. 2, and S103 and S112 of FIG. 3 are executed.

The electric characteristics at an arbitrary frequency, for example, can be used as the electric characteristics used in the present technique. The electric characteristics, for example, include impedance, conductance, admittance, capacitance, a dielectric constant, conductivity, a phase angle, quantities obtained by subjecting these characteristics to the electric quantity conversion, and the like. These electric characteristics can be used in combination of one kind or two or more kinds of these characteristics.

Examples

Hereinafter, the present technique will be described in more detail on the basis of test examples. It should be noted that the scope of the present technique is not narrowly interpreted by Examples which will be described below.

<Example 1>

Polymer beads (manufactured by Matsumoto Yushi-Seiyaku Co., Ltd., Matsumoto Microsphere) enclosing low-boiling hydrocarbon with acrylonitrile copolymer shell were used as the micro particles each having a lower density than that of the plasma, and polymer beads were dispersed to the PBS at the rate of 5%. The freeze-dried plasma was dissolved by the beads dispersing liquid to be made the sample for QC.

The various kinds of reagents such as aqueous solution of calcium, an intrinsic stimulation reagent, or an extrinsic stimulation reagent was put in a cartridge and the cartridge was set in the dielectric coagulometer. At this time, a reagent in which the concentrations of the intrinsic stimulation reagent and the extrinsic stimulation reagent were reduced to halves was also prepared to be made a model in the case where the activities of the reagents were halved. After the sample for QC containing the micro particles dispersing liquid was sufficiently mixed by inverting, the sample for QC was set in the dielectric coagulometer. Normally, after the sample for QC is dispensed within the cartridge which is kept warm at 37°C, the sample for QC is mixed with the various kinds of reagents within the cartridge by the stirring function, and is then measured.

Since the density of the micro particles used here is smaller than that of the plasma, the micro particles shall gradually rise within the cartridge being measured. Along with this, since the amount of insulating micro particles existing between the electrodes is reduced, the conductivity is temporally increased. On the other hand, as the coagulation of the plasma progresses by the aqueous solution of calcium or the stimulation reagent, the movement of the micro particles is limited, and the conductivity does not come to change. Therefore, since the coagulation speed of the plasma differs depending on the kind or concentration of reagent, this difference appears in the temporal change of the conductivity.

The suitable feature amount is extracted from the change in conductivity, and it is determined whether or not the value of the feature amount falls within a given range, thereby determining the state of the reagent contained in the cartridge, and the state of the apparatus.

FIG. 4 depicts the change in conductivity at 1 MHz as an example of the dielectric spectra obtained from the measurement described above. In the case where the coagulation reagent such as the aqueous solution of calcium was not added, since the coagulation of the plasma was not generated, the conductivity continued to rise. On the other hand, in the case where the aqueous solution of calcium was added, the coagulation of the plasma progressed during the measurement, and the movement of the micro particles was suppressed, so that the change in conductivity was not substantially observed after a lapse of approximately 1600 seconds. Similarly, in the case where the aqueous solution of calcium and the extrinsic stimulation reagent, or the aqueous solution of calcium and the intrinsic stimulation reagent were added, the coagulation of the plasma progressed more rapidly than the case where only the aqueous solution of calcium was added, so that the changes in conductivity were suppressed after a lapse of approximately 700 seconds or 1000 seconds, respectively. In addition, in the case where the concentration of the extrinsic stimulation reagent or the intrinsic stimulation reagent was halved as a model in the case where the activity of the reagent contained in the cartridge was reduced, the changes in conductivity were suppressed after a lapse of approximately 1000 seconds or 1400 seconds, respectively, which proved that this case was obviously more delayed than in the case of the normal concentrations.

The time until the change in conductivity is not observed is extracted as the feature amount in such a manner, and it is confirmed whether or not that time falls within the range previously set, thereby enabling the state (for example, the activity or the like) of the reagent contained in the cartridge to be determined.

<Example 2>

Although in Example 1 described above, the measurement was carried out at 37°C, the setting temperature of the apparatus was dared to be set at 30°C or 40°C which is lower or higher than that of the normal case, respectively, and the measurement was similarly carried out. Moreover, while the temperature was held at the normal temperature of 37°C, the measurement was carried out with the stirring function after dispensation of the sample for QC being stopped.

FIG. 5 depicts a change in conductivity at 1 MHz as an example of the dielectric spectra obtained by the measurement described above. In this case, when the aqueous solution of calcium, and the extrinsic stimulation reagent were added, at 37°C, the change in conductivity was suppressed after a lapse of approximately 700 seconds, whereas at 30°C, the change in conductivity was not observed after a lapse of approximately 600 seconds, and the magnitude of the change in conductivity from the start of the measurement was suppressed to approximately a half that in the case of 37°C. On the other hand, at 40°C, the change in conductivity was not observed after a lapse of approximately 1500 seconds, and the change in conductivity from the start of the measurement became approximately 7% to 8% larger than that in the case of 37°C. In addition, in the case where the stirring function was stopped, the magnitude of the change in conductivity from the start of the measurement was approximately 12% to 13% smaller than that in the case where the stirring was carried out.

In such a manner, the time for which there is no change in conductivity, or the magnitude of the change in conductivity from the start of the measurement is extracted as the feature amount. Then, it is confirmed whether or not the value of the feature amount falls within the range previously set, thereby enabling the state of the apparatus such as the temperature control, the stirring function or the like to also be confirmed.

<Comparative Example 1>

FIG. 6 depicts the change in conductivity at 1 MHz in the case where the freeze-dried plasma was dissolved without adding the micro particles, and the measurement was carried out with the dielectric coagulometer similarly to the case of Example 1 described above. In any case, the change in conductivity was small, and thus the clear change following the coagulation of the plasma was not observed. Although the change is observed for a lapse of approximately 200 seconds from the start of the measurement, this change is due to the temperature rising of the sample within the cartridge and has no relation to the coagulation.

It should be noted that although in Examples 1 and 2, the micro particles each having the smaller density than that of the plasma are used, the present technique is by no means limited to this as long as a change in beads distribution between the electrodes following the coagulation of the plasma can be observed. Thus, the beads having the larger density than that of the plasma may also be used. In addition, although FIG. 4 to FIG. 6 depict the temporal change of the conductivity at 1 MHz of the resulting dielectric spectra, the present technique is not especially limited to this frequency.

[Reference Signs List]

10: Electric characteristic measuring apparatus
1: Determination portion
2: Reference value setting portion
3: Measurement portion
4: Evaluation portion
5: Storage portion
6: Display portion
7: Reagent changing portion
8: Sample preparing portion
9: Apparatus setting portion

**Claims**

1. A method of verifying a measurement accuracy of an electric characteristic measuring apparatus by using the electric characteristic measuring apparatus to temporally measure electric characteristics of a control sample before temporally measuring electric characteristics of a blood sample, the method at least comprising:

   a reference range setting process for setting a reference range for measured electric characteristics pre-stored in an information storage medium, and
   a process for determining from temporally measured electric characteristics of the control sample and the reference range whether or not the measurement accuracy of the electric characteristic measurement apparatus is verified,
   wherein the control sample for measurement of electric characteristics at least includes a liquid containing a blood coagulation factor, and micro particles, and a density of the micro particles in the liquid is different from a mass density of the liquid containing the blood coagulation factor, each of the micro particles being different in conductivity or dielectric constant from the liquid containing the blood coagulation factor, the liquid being a dispersing liquid for dispersing the blood coagulation factor and/or the micro particles, and the method includes preparing the control sample for measurement of electric characteristics by adjusting the mass density of the micro particles in the liquid and a size of the micro particles in accordance a suitable measurement time, determining a change in conductivity or dielectric constant of the blood sample with respect to time, and determining a time until the conductivity or dielectric constant is suppressed to a threshold value, and/or an amount of change in conductivity or dielectric constant until the time at which the conductivity or dielectric constant is suppressed to the threshold value, the time and/or the amount of the change in the conductivity or dielectric constant being compared with the reference range to determine whether or not the measurement of the electric characteristic measurement apparatus is verified, wherein the measurement accuracy of the electric characteristic measurement apparatus is verified if the time and/or the amount of the change in the conductivity or dielectric constant fall within the reference range.

**2.** A method according to claim 1, comprising
deriving the blood coagulation factor from freeze-dried plasma.

**3.** A method according to claim 1 or claim 2, comprising
dispersing the micro particles in the dispersing liquid

**Patentansprüche**

**1.** Verfahren zur Bestätigung einer Messgenauigkeit einer Vorrichtung zur Messung elektrischer Eigenschaften durch Verwenden der Vorrichtung zur Messung elektrischer Eigenschaften zum zeitlichen Messen elektrischer Eigenschaften einer Kontrollprobe vor einem zeitlichen Messen elektrischer Eigenschaften einer Blutprobe, wobei das Verfahren zumindest umfasst:

einen Referenzbereichseinstellprozess zum Einstellen eines Referenzbereichs für gemessene elektrische Eigenschaften, die in einem Informationsspeichermedium vorgespeichert sind, und
einen Prozess zum Bestimmen aus zeitlich gemessenen elektrischen Eigenschaften der Kontrollprobe und dem Referenzbereich, ob die Messgenauigkeit der Vorrichtung zur Messung elektrischer Eigenschaften bestätigt wird oder nicht,
wobei die Kontrollprobe zur Messung elektrischer Eigenschaften mindestens eine Flüssigkeit mit einem Blutgerinnungsfaktor und Mikroteilchen umfasst, und eine Dichte der Mikroteilchen in der Flüssigkeit von einer Massendichte der Flüssigkeit mit dem Blutgerinnungsfaktor verschieden ist, jedes der Mikroteilchen eine von der Flüssigkeit mit dem Blutgerinnungsfaktor verschiedene Leifähigkeit oder dielektrische Konstante aufweist, die Flüssigkeit eine Dispergierflüssigkeit zum Dispergieren des Blutgerinnungsfaktors und/oder der Mikroteilchen ist, und das Verfahren umfasst:

Vorbereiten der Kontrollprobe zur Messung elektrischer Eigenschaften durch Anpassen der Massendichte der Mikroteilchen in der Flüssigkeit und einer Größe der Mikroteilchen gemäß einer geeigneten Messzeit,
Bestimmen einer Änderung der Leitfähigkeit oder der dielektrischen Konstante der Blutprobe in Bezug auf die Zeit, und
Bestimmen einer Zeit, bis die Leitfähigkeit oder die dielektrische Konstante auf einen Schwellenwert supprimiert wird, und/oder eines Betrags der Änderung der Leitfähigkeit oder der dielektrischen Konstante bis zur Zeit, zu welcher die Leitfähigkeit oder die dielektrische Konstante auf den Schwellenwert komprimiert wird, wobei die Zeit und/oder der Betrag der Änderung der Leitfähigkeit oder der dielektrischen Konstante mit dem Referenzbereich verglichen wird, um zu bestimmen, ob die Messung der Vorrichtung zur Messung elektrischer Eigenschaften bestätigt wird oder nicht, wobei die Messgenauigkeit der Vorrichtung zur Messung elektrischer Eigenschaften bestätigt wird, wenn die Zeit und/oder der Betrag der Änderung der Leitfähigkeit oder der dielektrischen Konstante in den Referenzbereich fällt.

**2.** Verfahren nach Anspruch 1, umfassend:
Gewinnen des Blutgerinnungsfaktors aus gefriergetrocknetem Plasma.

**3.** Verfahren nach Anspruch 1 oder Anspruch 2, umfassend:
Dispergieren der Mikroteilchen in der Dispergierflüssigkeit.

**Revendications**

1. Procédé de vérification d'une précision de mesure d'un appareil de mesure de caractéristiques électriques en utilisant l'appareil de mesure de caractéristiques électriques pour mesurer temporairement des caractéristiques électriques d'un échantillon de contrôle avant de mesurer temporairement des caractéristiques électriques d'un échantillon de sang, le procédé comprenant au moins :

   un procédé d'établissement de plage de référence pour établir une plage de référence pour des caractéristiques électriques mesurées préalablement stockées dans un support d'enregistrement d'informations, et
   un procédé pour déterminer à partir des caractéristiques électriques mesurées dans le temps de l'échantillon de contrôle et de la plage de référence si la précision de mesure de l'appareil de mesure de caractéristiques électriques est vérifiée ou non,
   dans lequel l'échantillon de contrôle pour la mesure de caractéristiques électriques comprend au moins un liquide contenant un facteur de coagulation du sang, et des microparticules, et une densité des microparticules dans le liquide est différente d'une densité massique du liquide contenant le facteur de coagulation du sang, chacune des microparticules ayant une conductivité ou une constante diélectrique différente de celle du liquide contenant le facteur de coagulation du sang, le liquide étant un liquide de dispersion pour disperser le facteur de coagulation du sang et/ou les microparticules, et le procédé comprend :

   la préparation de l'échantillon de contrôle pour une mesure de caractéristiques électriques en ajustant la densité massique des microparticules dans le liquide et une taille des microparticules en fonction d'un temps de mesure approprié,
   la détermination d'une variation de la conductivité ou de la constante diélectrique de l'échantillon de sang en fonction du temps, et
   la détermination d'un temps jusqu'à ce que la conductivité ou la constante diélectrique soit supprimée à une valeur de seuil, et/ou d'une quantité de changement de la conductivité ou de la constante diélectrique jusqu'au moment où la conductivité ou la constante diélectrique est supprimée à la valeur de seuil, le temps et/ou la quantité du changement de la conductivité ou de la constante diélectrique étant comparés à la plage de référence pour déterminer si la mesure de l'appareil de mesure de caractéristiques électriques est vérifiée ou non, dans lequel la précision de mesure de l'appareil de mesure de caractéristiques électriques est vérifiée si le temps et/ou la quantité de la variation de la conductivité ou de la constante diélectrique se situent dans la plage de référence.

2. Procédé selon la revendication 1, comprenant :
   la dérivation du facteur de coagulation du sang à partir de plasma lyophilisé.

3. Procédé selon la revendication 1 ou la revendication 2, comprenant :
   la dispersion des microparticules dans le liquide de dispersion.

# FIG.1

ELECTRIC CHARACTERISTIC MEASURING APPARATUS — 10

REFERENCE VALUE SETTING PORTION — 2

DETERMINATION PORTION — 1

STORAGE PORTION — 5

MEASUREMENT PORTION — 3

EVALUATION PORTION — 4

DISPLAY PORTION — 6

APPARATUS SETTING PORTION — 9

REAGENT CHANGING PORTION — 7

SAMPLE PREPARING PORTION — 8

# FIG.2

```
                          START

                          ↓

              PREPARE CONTROL SAMPLE  ∽S1

                          ↓

               SET REFERENCE VALUE  ∽S2          CHANGE CURRENT REAGENT  ∽S8
                                                     TO NEW REAGENT
                          ↓                              ↑

              MEASURE CONDUCTIVITY  ∽S3
                OF CONTROL SAMPLE             DISPLAY THAT ACTIVITY OF  ∽S7
                                                REAGENT IS ABNORMAL
                          ↓                              ↑

             EXTRACT FEATURE AMOUNT OF  ∽S4
           TEMPORAL CHANGE IN CONDUCTIVITY     RECORD THAT THERE IS
                                                  ABNORMALITY IN    ∽S6
                          ↓                     ACTIVITY OF REAGENT
                                                       ↑
                    ∽S5
                  DOES FEATURE
                  AMOUNT Tc FALL      No
                 WITHIN REFERENCE  ──────────→
                     RANGE?

                        Yes
                          ↓

              RECORD THAT ACTIVITY
              OF REAGENT IS NORMAL  ∽S9

                          ↓

            TEMPORALLY MEASURE ELECTRIC  ∽S10
          CHARACTERISTICS OF BLOOD SAMPLE

                          ↓

                         END
```

# FIG.3

START

↓

PREPARE CONTROL SAMPLE ⟿ S101

↓

SET REFERENCE VALUE ⟿ S102

↓

MEASURE CONDUCTIVITY OF CONTROL SAMPLE ⟿ S103

↓

EXTRACT FEATURE AMOUNT OF TEMPORAL CHANGE IN CONDUCTIVITY ⟿ S104

↓

DOES FEATURE AMOUNT Tc FALL WITHIN REFERENCE RANGE? ⟿ S105 — No →

RECORD THAT TEMPERATURE OF APPARATUS IS HIGH ⟿ S106 →

DISPLAY THAT SETTING OF APPARATUS IS ABNORMAL ⟿ S107 →

CHANGE SETTING OF APPARATUS ⟿ S108

Yes ↓

DOES FEATURE AMOUNT Dc FALL WITHIN REFERENCE RANGE? ⟿ S109 — No →

RECORD THAT TEMPERATURE OF APPARATUS IS LOW, OR STIRRING FAILURE ⟿ S110

Yes ↓

RECORD THAT SETTING OF APPARATUS IS NORMAL ⟿ S111

↓

TEMPORALLY MEASURE ELECTRIC CHARACTERISTICS OF BLOOD SAMPLE ⟿ S112

↓

END

# FIG.4

# FIG.5

# FIG.6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2008010058 A1, CHATELIER RONALD C [AU]; NEWMAN PET

- WO 2004109277 A1, FULLER JONATHAN A

**Non-patent literature cited in the description**

- **SASCHA MEYER DOS SANTOS et al.** A novel [mu]-fluidic whole blood coagulation assay based on Rayleigh surface acoustic waves as a point-of-care method to detect anticoagulants. *BIOMICROFLUID-ICS,* 01 September 2013, vol. 7 (5), 056502